# EUROPEAN PATENT SPECIFICATION

(11) **EP 2 915 526 B1**
(45) Date of publication and mention of the grant of the patent: **02.06.2021**
(21) Application number: 15158157.6
(22) Date of filing: 09.03.2015
(51) Int. Cl.: A61K 9/16

(54) **Pharmaceutical compositions comprising anagrelide**
Pharmazeutische Zusammensetzungen mit Anagrelid
Compositions pharmaceutiques comprenant de l'anagrelide

(30) Priority: 07.03.2014 EP 14158312; 07.03.2014 EP 14158317
(43) Date of publication of application: 09.09.2015
(73) Proprietor: Galenicum Health S.L.U., 08005 Barcelona (ES)
(72) Inventor: Arroyo Hidalgo, Sergio, 08005 Barcelona (ES); Puigvert Colomer, Marina, 08005 Barcelona (ES)

(56) References cited:
- EP-A1- 2 367 539
- US-A1- 2005 008 704
- US-A1- 2009 324 710

## Description

The present invention relates to pharmaceutical compositions comprising anagrelide hydrochloride monohydrate and to a process for the manufacture of said pharmaceutical compositions and to the use of said pharmaceutical compositions for the treatment of essential thrombocythemia.

### STATE OF THE ART

Essential thrombocythemia (ET) is an acquired myeloproliferative disorder characterised by an elevation of the platelet count above the normal range which in most laboratories is considered to be between 150,000 and 450,000 /microliter. The median age at diagnosis is 65 to 70 years, but the disease can occur at any age. ET is a very rare disease with an annual incidence of about 2.5 cases/100,000/year. In general, increasing platelet counts, increasing age and additional risk factors such as hypercholesterolaemia and/or diabetes mellitus which lead to vascular alterations are associated with an increased risk for thromboembolic complications.

In principle the aim of a ET treatment should be (a) curing the underlying disease, or (b) a long-term decrease of the platelet number to normal or near to normal values to reduce clinical complications (for example, below 450,000 - 600,000/microliter). The underlying cause of ET is currently unknown and it can therefore not be cured with current treatment modalities. The therapeutic approach is therefore to find drugs to decrease elevated platelet counts and in doing so reduce the incidence or prevent clinical complications associated with elevated platelet counts.

Xagrid® is a marketed medicinal product indicated for the reduction of elevated platelet counts in at risk ET patients who are intolerant to their current therapy or whose elevated platelet counts are not reduced to an acceptable level by their current therapy. An at risk ET patient is defined by one or more of the following features: more than 60 years of age or a platelet count above 1,000 x 10⁹/l or a history of thrombo-haemorrhagic events. Xagrid® is in the form of hard gelatine capsules, each of which contains anagrelide hydrochloride equivalent to 0.5 mg of anagrelide.

Anagrelide was first disclosed in US 3,932,407 and is a phosphodiesterase inhibitor. This imidazoquinazoline was originally developed as an inhibitor of platelet aggregation but subsequently found to have value as a platelet-lowering agent for the treatment of patients suffering ET. Anagrelide is also known as 6,7-dichloro-1,5-dihydroimidazo[2,1-b]quinazolin-2(3H)-one. The empirical formula of anagrelide hydrochloride monohydrate is C10H7Cl2N3O.HCl.H2O and the compound has a molecular weight of 310.55 g/mol. The structural formula of anagrelide hydrochloride monohydrate is (I):

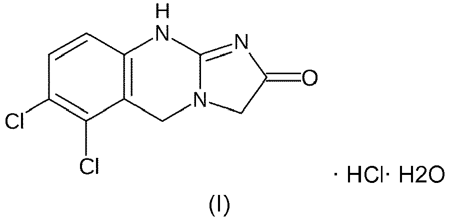

WO 2010/063824 and EP 2367539 B1 relate to anagrelide formulations with non-immediate release. US 2005/008704 A1 relates to formulations with enhanced solubility comprising anagrelide and polyethylene glycol. WO 02/058676 and US 2003/0158261 relate to anagrelide sustained release formulations with a pH independent release profile.

### DESCRIPTION OF THE INVENTION

The present invention provides a stable pharmaceutical composition of anagrelide hydrochloride monohydrate. The pharmaceutical compositions of the present invention have a fast onset, which permits a higher bioavailability. The pharmaceutical composition according to the present invention offers the advantage of a low content of total impurities originating from the decomposition of anagrelide hydrochloride monohydrate. The scope of the invention is defined by the claims. Any references in the description to methods of treatment refer to the products (compounds, pharmaceutical compositions and medicaments) of the present invention for use in a method for treatment of the human or animal body by therapy or diagnosis. Any "embodiment" or "example" which is disclosed in the description but is not covered by the claims should be considered as presented for illustrative purpose only.

The inventors have found that a certain interval of concentration of the active agent in a granulate or in a pharmaceutical composition of said active agent, results in a pharmaceutical composition with immediate release dissolution profile and with reduced percentage of impurities. Also, the pharmaceutical composition of the present invention can be easily filled into hard capsules, because of the flowability of said pharmaceutical composition and of the reduced amount in each capsule.

The present invention relates to a pharmaceutical composition according to claim 1 comprising a granulate comprising an active agent, wherein the active agent is anagrelide hydrochloride monohydrate. The active agent has a particle size volume distribution with D50 between 0.4 and 10 microns, and a D90 between 3 and 20 microns, when measured by laser diffraction analysis; wherein said granulate has a particle size volume distribution with a D90 between 50 and 800 microns, preferably with a D90 between 70 and 700 microns, more preferably with a D90 between 80 and 600 microns, when measured by sieve analysis; and wherein at least 85 % of the active agent of the pharmaceutical granulate dissolves in 15 minutes in a paddle dissolution test apparatus, at 50 rpm with a HCI solution at pH 1.2 as dissolution medium and a volume of 900 ml per vessel.

The term "granulate" or "granular component" as used herein refers to the part of the pharmaceutical composition that has been obtained by granulating a powder into larger particles herein called granules. Said granulation can be either wet granulation or dry granulation. Preferably, the granulate of the present invention is obtained by wet granulation. The term "extragranular component" as used herein refers to the set of ingredients in the pharmaceutical composition which have not been granulated and which do not form part of the granular component.

In a preferred embodiment, at least 90 % of the active agent of the pharmaceutical granulate dissolves in 15 minutes in a paddle dissolution test apparatus, at 50 rpm with a HCI solution at pH 1.2 as dissolution medium and a volume of 900 ml per vessel. In a preferred embodiment, the active agent has a particle size volume distribution with a D50 between 0.5 and 6.0 microns and a D90 between 4.0 and 12.0 microns, preferably has a D50 between 0.8 and 5.0 microns and a D90 between 4.5 and 11.0 microns, more preferably has a D50 between 1.0 and 4.0 microns and a D90 between 5.0 and 10.0 microns, when measured by laser diffraction analysis.

In a preferred embodiment, the amount of active agent is between 0.43 and 2.0 % by weight in respect of the total amount of the pharmaceutical granulate. In a preferred embodiment, the amount of active agent is between 0.55 and 0.77 % by weight in respect of the total amount of the pharmaceutical composition.

The present invention relates to a pharmaceutical composition comprising the pharmaceutical granulate comprising anagrelide hydrochloride monohydrate. Preferably, it relates to a pharmaceutical composition consisting essentially of the pharmaceutical granulate comprising anagrelide hydrochloride monohydrate.

The present invention relates to a pharmaceutical composition comprising an active agent, wherein the active agent is anagrelide hydrochloride monohydrate, wherein the amount of active agent is between 0.42 and 1.59 % by weight in respect of the total amount of the pharmaceutical composition, and wherein at least 85 % of the active agent of the pharmaceutical composition dissolves in 15 minutes in a paddle dissolution test apparatus, at 50 rpm with a HCI solution at pH 1.2 as dissolution medium and a volume of 900 ml per vessel. In a preferred embodiment, the amount of active agent is between 0.43 and 0.53 % by weight in respect of the total amount of the pharmaceutical composition. In a preferred embodiment, at least 90 % of the active agent of the pharmaceutical composition dissolves in 15 minutes in a paddle dissolution test apparatus, at 50 rpm with a HCl solution at pH 1.2 as dissolution medium and a volume of 900 ml per vessel. The pharmaceutical composition comprises a granulate which contains the active agent.

In a preferred embodiment, the pharmaceutical composition comprises a granulate containing the active agent and wherein said granulate has a particle size volume distribution with a D90 between 50 and 800 microns, when measured by sieve analysis. In a preferred embodiment, the pharmaceutical composition comprises a granulate containing the active agent and wherein said granulate has a particle size volume distribution with a D90 between 70 and 700 microns, when measured by sieve analysis. In a preferred embodiment, the pharmaceutical composition comprises a granulate containing the active agent and wherein said granulate has a particle size volume distribution with a D90 between 80 and 600 microns, when measured by sieve analysis.

In a preferred embodiment, the pharmaceutical composition comprises at least one lubricant, preferably the lubricant is magnesium stearate. In a preferred embodiment, the lubricant is extragranular. In a preferred embodiment, the amount of lubricant ranges from 0.3 to 1.2 % by weight in respect of the total amount of the pharmaceutical composition, preferably ranges from 0.4 to 0.6 % by weight in respect of the total amount of the pharmaceutical composition. The flowability of the pharmaceutical composition is good when the lubricant is extragranular. Also, the dissolution profile corresponds to an immediate release when the lubricant is extragranular. This amount of lubricant contributes to the immediate release dissolution profile and also entails an easy process for the filling of the capsules with the pharmaceutical composition of the invention.

The term "pharmaceutically acceptable" indicates that the substance or composition must be compatible chemically and/or toxicologically, with the other ingredients comprising a formulation, the mammal being treated therewith, and/or the route of administration of the composition.

As used herein, "lubricant" means a substance that reduces the friction both between the components of the composition of the present invention, and between the composition and the surfaces of the apparatus used to prepare said composition.

In a preferred embodiment, the active agent has a particle size volume distribution with D50 between 1 and 10 microns, and a D90 between 3 and 20 microns, preferably a D50 between 0.5 and 6.0 microns and a D90 between 4.0 and 12.0 microns, when measured by laser diffraction analysis. In a preferred embodiment, the active agent has a particle size volume distribution with a D50 between 0.8 and 5.0 microns and a D90 between 4.5 and 11.0 microns, when measured by laser diffraction analysis. In a preferred embodiment, the active agent has a particle size volume distribution with a D50 between 1.0 and 4.0 microns and a D90 between 5.0 and 10.0 microns, when measured by laser diffraction analysis.

In a preferred embodiment, the pharmaceutical composition comprises an amount of active agent per hard capsule between 0.45 and 1.30 mg, preferably between 0.48 and 1.27 mg.

In a preferred embodiment, the pharmaceutical composition comprises an amount of active agent per hard capsule equivalent to 0.5 mg or 1 mg of the active agent as free base.

The pharmaceutical composition comprises a filler. The filler is a mixture of anhydrous lactose, lactose monohydrate and microcrystalline cellulose. In a preferred embodiment, the extragranular filler is anhydrous lactose. The inventors have found that when anhydrous lactose is used as extragranular filler, the pharmaceutical composition has a good stability and a low amount of impurities.

The term "filler" as used herein refers to pharmaceutically acceptable excipients which are added to the bulk volume of the active agent making up the solid composition. As a result, the size of the solid composition increases, which makes its size suitable for handling. Fillers are convenient when the dose of drug per solid composition is low and the solid composition would otherwise be too small.

The pharmaceutical composition comprises less than 60 mg of lactose monohydrate.

In a preferred embodiment, the filler is both intragranular and extragranular.

In a preferred embodiment, the pharmaceutical composition is prepared by wet granulation.

In a preferred embodiment, the pharmaceutical composition comprises granules containing between 0.58 and 0.64 mg of anagrelide hydrochloride monohydrate, and an extragranular lubricant, preferably magnesium stearate in an amount ranging from 0.3 to 1.2 % by weight in respect of the total amount of the pharmaceutical composition.

In a preferred embodiment, the pharmaceutical composition comprises a granulate having a particle size volume distribution with a D90 between 70 and 700 microns, wherein said granulate comprises between 0.48 and 1.27 mg of active agent, and wherein the pharmaceutical composition comprises less than 60 mg of lactose monohydrate.

In a preferred embodiment, the pharmaceutical composition is in form of a hard capsule.

A further aspect of the present invention relates to a hard capsule comprising between 80 and 150 mg of a pharmaceutical composition comprising a granulate comprising an active agent, wherein the active agent is anagrelide hydrochloride monohydrate; wherein at least 85 % of the active agent of the pharmaceutical composition, preferably at least 90 % of the active agent of the pharmaceutical composition, dissolves in 15 minutes in a paddle dissolution test apparatus, at 50 rpm with a HCI solution at pH 1.2 as dissolution medium and a volume of 900 ml per vessel; and wherein the pharmaceutical composition comprises an amount of active agent per hard capsule between 0.45 and 1.30 mg, preferably between 0.48 and 1.27 mg. Preferably, the hard capsule comprises between 90 and 145 mg of the pharmaceutical composition, more preferably comprises between 100 and 140 mg of the pharmaceutical composition. In a preferred embodiment, said pharmaceutical composition comprises an amount of active agent per hard capsule equivalent to 0.5 mg or 1 mg of the active agent as free base. Preferably, the unit dose of the hard capsule is equivalent to 0.5 mg or 1 mg of the active agent as free base. In another preferred embodiment, the hard capsule is a hard gelatine capsule.

As used herein, the term "unit dose" or "unit dosage" refers to a physically discrete unit that contains a predetermined quantity of active agent calculated to produce a desired therapeutic effect. The unit dose or unit dosage may be in the form of a vial, tablet, capsule, sachet, etc. referred to herein as a "unit dosage form".

In a preferred embodiment, the hard capsule comprises between 120 and 140 mg of a pharmaceutical composition comprising granules containing between 0.58 and 0.64 mg of anagrelide hydrochloride monohydrate, and an extragranular lubricant, preferably magnesium stearate in an amount ranging from 0.3 to 1.2 % by weight in respect of the total amount of the pharmaceutical composition.

In a preferred embodiment, the hard capsule comprises a pharmaceutical composition comprising a granulate having a particle size volume distribution with a D90 between 70 and 700 microns, wherein said granulate comprises between 0.48 and 1.27 mg of active agent, and wherein the pharmaceutical composition comprises less than 60 mg of lactose monohydrate.

A further aspect relates to anagrelide hydrochloride monohydrate having a particle size volume distribution with D50 between 0.4 and 10 microns, and a D90 between 3 and 20 microns, preferably with a D50 between 0.5 and 6.0 microns and a D90 between 4.0 and 12.0 microns, more preferably with a D50 between 0.8 and 5.0 microns and a D90 between 4.5 and 11.0 microns, even more preferably with a D50 between 1.0 and 4.0 microns and a D90 between 5.0 and 10.0 microns, when measured by laser diffraction analysis.

A further aspect relates to a pharmaceutical batch comprising at least 20,000 hard capsules as disclosed in the present invention. Preferably, the pharmaceutical batch comprises at least 50,000 hard capsules. More preferably, the pharmaceutical batch comprises at least 100,000 hard capsules. In a preferred embodiment, the content of the active agent is uniform. In a preferred embodiment, the hard capsules are packaged in light-protected bottles with desiccant.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active agent in the compositions of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active agent" refers to a therapeutically active compound, as well as any prodrugs thereof and pharmaceutically acceptable salts, hydrates and solvates of the compound and the prodrugs.

For the release of a pharmaceutical batch the distribution of the active agent in the pharmaceutical compositions has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

A further aspect relates to a process for manufacturing a pharmaceutical granulate of the invention, comprising the following steps:
i) weighing, optionally sieving and mixing the active agent and optionally at least one pharmaceutically acceptable excipient;
ii) wet granulating the mix obtained in step (i);
iii) drying the granulate obtained in step (ii); and
iv) optionally sieving the granulate obtained in step (iii).

In one embodiment of the present invention, the wet granulation of step (ii) is performed in a fluid bed granulator, a high shear granulators, or a one pot processor.

A further aspect relates to the pharmaceutical granulate manufactured by the process of the foregoing aspect.

A further aspect of the present invention relates to a process for manufacturing a pharmaceutical composition of the invention, comprising the following steps:
i) weighing, optionally sieving and mixing the active agent and optionally at least one pharmaceutically acceptable excipient;
ii) wet granulating the mix obtained in step (i);
iii) drying the granulate obtained in step (ii);
iv) optionally sieving the granulate obtained in step (iii); and
v) optionally adding at least one extragranular pharmaceutically acceptable excipient to the granulate obtained in step (iii) or (iv).

A further aspect of the present invention relates to the pharmaceutical composition manufactured by the process of the foregoing aspect.

A further aspect of the present invention relates to a process for manufacturing a hard capsule of the invention, comprising the following steps:
i) weighing, optionally sieving and mixing the active agent and optionally at least one pharmaceutically acceptable excipient;
ii) wet granulating the mix obtained in step (i);
iii) drying the granulate obtained in step (ii);
iv) optionally sieving the granulate obtained in step (iii);
v) optionally adding at least one extragranular pharmaceutically acceptable excipient to the granulate obtained in step (iii) or (iv); and
vi) filling the mix obtained in step (i), or the granulate obtained in step (iii) or in step (iv), or the mix obtained in step (v), into hard capsules.

A further aspect of the present invention relates to the hard capsule manufactured by the process of the foregoing aspect.

A further aspect of the present invention relates to a process for manufacturing a pharmaceutical batch of a hard capsule of the invention, comprising the following steps:
i) manufacturing a test hard capsule;
ii) checking the stability and dissolution profile of the test hard capsule of step (i); and
iii) manufacturing a pharmaceutical batch by the same manufacturing process used to prepare the test hard capsule in step (i) provided that the test hard capsule is stable and has an immediate release dissolution profile; and
iv) optionally, packaging the pharmaceutical batch manufactured in step (iii) preferably in bottles.

In a preferred embodiment of the process of the foregoing aspect, the stability checked in step (ii) is the stability after at least one day at 40 °C and 75 % relative humidity.

A further aspect of the present invention relates to a pharmaceutical batch manufactured by the process of the foregoing aspect.

A further aspect of the present invention relates to a process for the validation of a pharmaceutical batch of the invention, comprising the following steps:
i) manufacturing the pharmaceutical batch;
ii) checking the uniformity of the active agent content; and
iii) validating the batch only if the content is uniform.

A further aspect of the present invention relates to the pharmaceutical granulate or the pharmaceutical composition of the invention; or the hard capsule or the pharmaceutical batch of the invention, for use in the treatment of essential thrombocythemia. Preferably, for the reduction of elevated platelet counts in essential thrombocythemia patients of more than 60 years of age or having a platelet count above 1,000 x 10⁹/l or having a history of thrombo-hemorrhagic events.

A further aspect of the present invention relates to a packaged pharmaceutical composition in form of a bottle containing at least five hard capsules, preferably wherein said bottle is light-resistant and comprises a desiccant; or a cardboard box with a patient information leaflet comprising at least one bottle containing at least five hard capsules.

Preferably, the bottle has a child-resistant closure.

The pharmaceutical compositions, the granulate and the hard capsules of the present invention are stable. The term "stable" as used herein refers to a pharmaceutical composition comprising anagrelide wherein the total content of impurities originating from the decomposition of anagrelide does not exceed 5 % area, preferably 3 % area, more preferably 2 % area and most preferably 1.5 % area determined as specified in the US Pharmacopoeia official monograph for anagrelide capsules.

An immediate release pharmaceutical composition as herein disclosed has to be understood as a pharmaceutical composition where at least 85 % of the active agent is released in 15 minutes. In a preferred embodiment, the pharmaceutical composition as herein disclosed releases at least 90 % of the active agent in 15 minutes. The dissolution test for an immediate release pharmaceutical composition comprising the active agent as herein disclosed is performed as disclosed in the examples.

All percentages, parts, and ratios herein are by weight unless specifically noted otherwise. As used herein, the term "about" refers preferably to a range that is ±10 %, preferably ±5 %, or more preferably ±1 % of a value with which the term is associated

Unless otherwise indicated, all the analysis methods are carried out according to the European Pharmacopoeia 7th edition.

### DESCRIPTION OF THE FIGURES

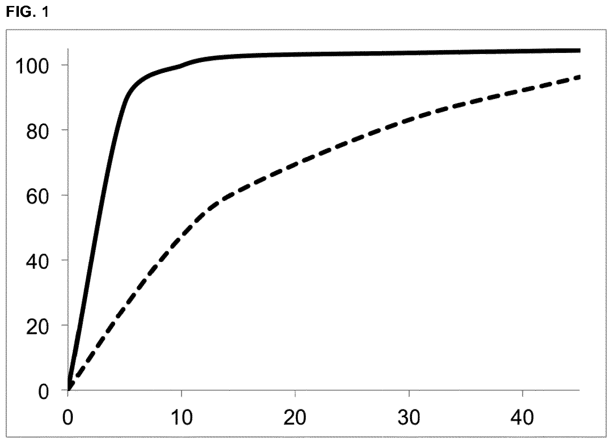

Figure 1 depicts the immediate release dissolution profile (% dissolved vs time in minutes) of capsules of example 2b (solid line) and the dissolution profile of capsules as disclosed in WO 2010/063824 (dashed line). The dissolution test is performed as explained in the examples.

### EXAMPLES

The following examples illustrate various embodiments of the invention and are not intended to limit the invention in any way.

### Preparatory Example 1. Process to obtain anagrelide hydrochloride monohydrate.

A mixture of N-(2-amino-5,6-dichlorobenzyl)glycine ethyl ester (2.77 g, 0.01 mol) and 3,5-dimethyl-pyrazole-1-carboxamidine nitrate (2.01 g, 0.01 mol) in absolute ethanol (30 ml) was heated at reflux for 24 hours. The mixture was filtered and the product was washed successively with ethanol, acetone and ether to yield the title compound (1.02 g, 40 %).

Anal. Calcd for C10H7Cl2N3O: C, 46.90; H, 2.76; N, 16.41. Found: C, 46.50; H, 2.76; N, 16.82.

6,7-Dichloro-1,5-dihydroimidazo[2,1-b]-quinazolin-2(3H)-one (94.0 g, 0.367 mol) was added to 2.82 l of boiling methanol. To the suspension was added 77 ml (0.92 mol) of concentrated hydrochloric acid. A nearly complete solution resulted. Darco G-60 was added, the mixture was boiled for ca. 3 minutes and then filtered. The filtrate was heated to boiling, then cooled slightly, and 1.9 l of ether added cautiously. The mixture was stirred at ambient temperature for 2 hours and the solid was filtered off (Crop A). Some material tended to crystallize in the funnel during the charcoal treatment step; the charcoal and solids therefore were suspended in hot methanol and filtered. This filtrate was combined with the filtrate from Crop A, and the combined filtrates were concentrated in vacuo to about 1.3 l and heated to boiling to effect complete solution. After filtration and slight cooling, 800 ml of ether were added and the mixture was stirred at room temperature for 1 hour. The resulting solid was filtered off (Crop B). Chromatography showed both crops to be of very high purity. They were combined and dried in vacuo (ca. 1 mm Hg) overnight. Total weight of product was 88.1 g. Karl Fischer analysis indicated 2.27 % water. The material was placed in a desiccator over saturated aqueous Ca(NO3)2 (ca. 50 % relative humidity) and analysed for water daily. After 4 days analysis showed 5.64 % water (5.80 % theoretical for monohydrate). The weight of product was 91.9 g (80.6% of theoretical).

### Example 2. Granulate comprising anagrelide hydrochloride monohydrate.

Granulates of the compositions of table 1 are prepared by wet granulation, where the active agent and 2.61 g of the crospovidone are intragranular and the rest of the crospovidone as well as the magnesium stearate are extragranular.

The manufacturing process of the granulates of examples 2a, 2b and 2c and of hard capsules comprising said granulate is briefly described below:
a. The active agent, the lactose monohydrate, the microcrystalline cellulose and part of the crospovidone were weighed and sifted through 1 mm sieve.
b. The components of step (a) were added to a high shear granulator and mixed for 15 minutes.
c. The povidone was dissolved in the purified water with gentle shaking and at room temperature.
d. The solution obtained in step (c) was added to the blend obtained in step (b) and blended.
e. The blend obtained in step (d) was granulated.
f. The granules were dried in a fluidized bed dryer with a maximum air temperature of 45 °C, until the water content was under 5 % (Loss on drying at 105 °C).
g. The dried granulate was sieved through a 1 mm sieve and mixed.
h. The rest of the crospovidone, the magnesium stearate and optionally the anhydrous lactose were weighed and were mixed with the dried granulate obtained in step (g), before being encapsulated in hard capsules.
i. The capsules were then packed in plastic bottles with a desiccant cartridge (silica gel). Each bottle was filled with 100 capsules.

**Table 1. Compositions of the granulates.**

| Component | Ex. 2a | Ex. 2b | Ex. 2c |
|---|---|---|---|
| active agent* (g) | 0.61 | 0.61 | 0.61 |
| anhydrous lactose (g) | - | 65.80 | 48.61 |
| lactose monohydrate (g) | 92.36 | 53.70 | 53.70 |
| povidone (g) | 4.65 | 4.65 | 4.65 |
| crospovidone (g) | 4.35 | 4.35 | 4.35 |
| microcrystalline cellulose (g) | 17.43 | 20.14 | 17.43 |
| purified water** (g) | 26.35 | 20.00 | 20.00 |
| magnesium stearate (g) | 0.60 | 0.75 | 0.65 |
| Total (g) | 120.00 | 150.00 | 130.00 |

| | | | |
|---|---|---|---|
| * equivalent to 0.5 mg anagrelide free base. ** removed during drying. | | | |

After preparing the granulates of examples 2a and 2b as explained above, around 1,000 hard gelatine capsules were filled with 120 mg of each granulate. The capsules were packaged in light resistant HDPE bottles with child-resistant closure and desiccant (100 capsules per bottle).

### Example 3. Assay and content uniformity.

The amount of active agent per capsule was analysed by HPLC. The capsules of example 2 showed very good assay and content uniformity. For example, capsules of example 2a had an assay of 99.75 % ± 0.87 %.

### Example 4. Stability.

The impurity content of the capsules of example 2 were analysed by HPLC. Several batches were prepared of the composition of example 2a. The impurity content results of tables 2 and 3 are from different batches of the composition of example 2a.

**Table 2 summarizes the impurity content as percentage in respect of the total amount of the active agent.**

| Example 2a | time 0 | 7 months at RT* |
|---|---|---|
| Impurity B | 0.37 | 0.52 |
| Total impurities | 0.50 | 0.72 |

| | | |
|---|---|---|
| * RT: room temperature. | | |

**Table 3 summarizes the impurity content as percentage in respect of the total amount of the active agent after 1.5 months at the following conditions:**

| Example 2a | 25 °C / 60 % RH* | 30 °C / 65 % RH* | 30 °C / 75 % RH* | 45 °C / 75 % RH* |
|---|---|---|---|---|
| Impurity B | < 0.05 | 0.05 | 0.05 | 0.05 |
| Total impurities | 0.08 | 0.13 | 0.13 | 0.13 |

| | | | | |
|---|---|---|---|---|
| * RH: relative humidity. | | | | |

### Example 5. Dissolution profiles.

The dissolution profiles were analysed in the following conditions: USP Apparatus 2 (paddles); speed: 50 rpm; medium: HCI pH 1.2; volume: 900 ml per vessel; wavelength: 274 nm, for capsules at t=0 and after 53 days of stability at 25 °C / 60 % RH; at 30 °C / 60 % RH; and at 40 °C / 75 % RH. The dissolution profiles of the capsules of all the examples above are homogeneous and stable, and corresponded to an immediate release composition, with more than 85% of the active agent dissolved after 15 minutes.

### Example 6. Particle size distribution.

The particle size distribution of the active agent was analysed by laser diffraction spectroscopy using a Malvern Mastersizer 2000 particle size analyzer. The anagrelide used for the compositions of examples 2a and 2b had a particle size volume distribution with a D90 smaller than 10 microns, between 6 and 8 microns, and a D50 smaller than 6 microns, between 2 and 4 microns.

The particle size distribution of the granulates was analysed by sieve analysis using a mechanical shaker. The granulates of the examples showed a D90 between 100 and 500 microns.

Examples 7-12 are reference examples which are not covered by the claims.

### Example 7. Pharmaceutical powder comprising anagrelide.

| Component | Ex. 7 |
|---|---|
| active agent* (g) | 0.61 |
| anhydrous lactose (g) | 92.36 |
| povidone (g) | 4.65 |
| crospovidone (g) | 4.35 |
| microcrystalline cellulose (g) | 17.43 |
| magnesium stearate (g) | 0.60 |
| Total (g) | 120.00 |

| | |
|---|---|
| * equivalent to 0.5 mg anagrelide free base. | |

The pharmaceutical powder comprising anagrelide as active agent was prepared as follows:
a. The active agent was weighed, mixed with about a third of the lactose and sieved through a 1 mm sieved, conducting three progressive dilutions and mixing the active agent with the total amount of lactose.
b. The povidone, crospovidone and crystalline microcellulose were weighed, sieved, added to the mixture of step (a) and mixed. The premix was sieved through 1 mm sieve and mixed again.
c. The magnesium stearate was weighed, sieved and added to the premix from step (b). The resultant mix was sieved through a 1 mm sieve and mix during 5 minutes to obtain the final mixture, which was encapsulated in hard capsules.
d. The capsules comprising the pharmaceutical powder were packed in plastic bottles with or without a desiccant cartridge (silica gel). Each bottle was filled with 100 capsules.

### Example 8. Pharmaceutical dry granulate comprising anagrelide.

Dry granulates comprising anagrelide were prepared based on the composition of example 7, as follows:
a. The active agent was weighed, mixed with about a third of the lactose and sieved through a 1 mm sieved, conducting three progressive dilutions and mixing the active agent with the total amount of lactose.
b. The povidone, crospovidone and crystalline microcellulose were weighed, sieved, added to the mixture of step (a) and mixed. The premix was sieved through 1 mm sieve and mixed again.
c. The magnesium stearate was weighed, sieved and added to the premix from step (b). The resultant mix was sieved through a 1 mm sieve and mix during 5 minutes to obtain the final mixture.
d. The final mixture was compacted, milled and sieved through a 3 mm sieve. Then, the dry granulate was sieved through a 1.25 mm sieve and mixed.
e. The dry granulate obtained in step (d) was encapsulated in hard capsules.
f. The capsules comprising the granulate were packed in plastic bottles with or without a desiccant cartridge (silica gel). Each bottle was filled with 100 capsules.

After preparing the pharmaceutical powder and the dry granulate as explained above, around 1,000 hard gelatine capsules were filled with 120 mg of each granulate. The capsules were packaged in light resistant HDPE bottles with child-resistant closure (100 capsules per bottle).

### Example 9. Assay and content uniformity.

The amount of active agent per capsule was analysed by HPLC. The capsules of examples 7 and 8 showed very good assay and content uniformity. For example, capsules of example 7 had a content uniformity of 102.3 % (RSD 2.1 %).

### Example 10. Stability and water content.

Capsules of examples 7 and 8 were packaged in bottles as described above and were subjected to stability tests at times 0 and 1 month or 53 days at 25 °C/60 % relative humidity (RH), 30 °C/65 % RH, 30 °C/75 % RH and 40 °C/75 % RH. The impurity content was assessed by HPLC. The water content was analysed by Karl Fisher titration.

**Table 1. Impurity content (%) and water content (%) of the capsules of example 7:**

| Ex. 7 | t=0 | 53 days 25°C/60%HR | 53 days 30°C/65%HR | 53 days 30°C/75%HR | 53 days 40°C/75%HR |
|---|---|---|---|---|---|
| Impurity B | 0.3 | 0.3 | 0.3 | 0.3 | 0.3 |
| Unknown impurities | < 0.1 | < 0.1 | 0.1 | 0.1 | 0.1 |
| Total impurities | 0.3 | 0.3 | 0.4 | 0.4 | 0.4 |
| Water content | 1.7 | 1.6 | 1.6 | 1.6 | 1.7 |

**Table 2. Impurity content (%) and water content (%) of the capsules of example 8:**

| Ex. 8 | t=0 | 1m 25°C/60%HR | 1m 30°C/65%HR | 1m 30°C/75%HR | 1m 40°C/75%HR |
|---|---|---|---|---|---|
| Impurity B | 0.4 | 0.4 | 0.4 | 0.4 | 0.3 |
| Unknown impurities | < 0.1 | < 0.1 | < 0.1 | < 0.1 | < 0.1 |
| Total impurities | 0.5 | 0.5 | 0.5 | 0.4 | 0.4 |
| Water content | 1.7 | 1.6 | 1.6 | 1.6 | 1.7 |

### Example 11. Dissolution profiles.

The dissolution profiles were analysed following the method as specified in the US Pharmacopoeia official monograph for anagrelide capsules (Apparatus 1 (basket), speed: 100 rpm, vessel volume: 900 ml, dissolution medium: 0.1 N HCI, wavelength: 274 nm), for capsules at t=0 and after 53 days of stability at 25 °C / 60 % RH; at 30 °C / 60 % RH; and at 40 °C / 75 % RH. The dissolution profiles of the capsules of examples 7 and 8 are homogeneous and stable, and correspond to an immediate release composition, with more than 85% of the active agent dissolved at time 15 minutes.

### Example 12. Particle size distribution.

The particle size distribution was analysed by laser diffraction spectroscopy using a Malvern Mastersizer 2000 particle size analyser. The anagrelide used for the compositions of examples 7 and 8 had a particle size volume distributions with a D90 smaller than 10 microns, between 6 and 8 microns, and a D50 smaller than 6 microns, between 1.5 and 4 microns.

## Claims

1. A pharmaceutical composition comprising a granulate comprising an active agent, wherein the active agent is anagrelide hydrochloride monohydrate, wherein the amount of active agent is between 0.42 and 1.59 % by weight in respect of the total amount of the pharmaceutical composition, preferably between 0.43 and 0.53 % by weight in respect of the total amount of the pharmaceutical composition, wherein at least 85 %, preferably at least 90 % of the active agent of the pharmaceutical composition dissolves in 15 minutes in a paddle dissolution test apparatus, at 50 rpm with a HCI solution at pH 1.2 as dissolution medium and a volume of 900 ml per vessel, wherein the pharmaceutical composition comprises a filler, wherein the filler is a mixture of anhydrous lactose, lactose monohydrate and microcrystalline cellulose; and wherein the pharmaceutical composition comprises less than 60 mg of lactose monohydrate.

2. The pharmaceutical composition according to the preceding claim, wherein the pharmaceutical composition comprises a granulate which contains the active agent, wherein said granulate has a particle size volume distribution with a D90 between 70 and 700 microns, when measured by sieve analysis.

3. The pharmaceutical composition according to any one of the two preceding claims, wherein the pharmaceutical composition comprises at least one lubricant, preferably the lubricant is magnesium stearate and/or the lubricant is preferably extragranular.

4. The pharmaceutical composition according to any one of the preceding claims, wherein the pharmaceutical composition is prepared by wet granulation and/or is in form of a hard capsule.

5. The pharmaceutical composition according to the preceding claim, wherein the amount of lubricant ranges from 0.3 to 1.2 % by weight in respect of the total amount of the pharmaceutical composition, preferably ranges from 0.4 to 0.6 % by weight in respect of the total amount of the pharmaceutical composition, and/or wherein the pharmaceutical composition comprises an amount of active agent per hard capsule between 0.45 and 1.30 mg, preferably between 0.48 and 1.27 mg, more preferably the pharmaceutical composition comprises an amount of active agent per hard capsule equivalent to 0.5 mg or 1 mg of the active agent as free base.

6. A pharmaceutical batch comprising at least 20,000, preferably at least 50,000, more preferably at least 100,000 hard capsules as defined in any one of claims 4 or 5.

7. A process for preparing a pharmaceutical composition according to claims 1 to 4, comprising the following steps:
i) weighing, optionally sieving and mixing the active agent and optionally at least one pharmaceutically acceptable excipient;
ii) wet granulating the mix obtained in step (i);
iii) drying the granulate obtained in step (ii); and
iv) optionally sieving the granulate obtained in step (iii).

8. A packaged pharmaceutical composition in form of a bottle containing at least five hard capsules as defined in any one of claims 4 to 5, preferably wherein said bottle is light-resistant and comprises a desiccant.

9. The pharmaceutical composition according to any one of claims 1 to 5 or the pharmaceutical batch according to claim 6 for use in the treatment of essential thrombocythemia.

10. A process for manufacturing a hard capsule according to any one of claims 4 or 5, comprising the following steps:
i) weighing, optionally sieving and mixing the active agent and optionally at least one pharmaceutically acceptable excipient;
ii) wet granulating the mix obtained in step (i);
iii) drying the granulate obtained in step (ii);
iv) optionally sieving the granulate obtained in step (iii);
v) optionally adding at least one extragranular pharmaceutically acceptable excipient to the granulate obtained in step (iii) or (iv); and
vi) filling the mix obtained in step (i), or the granulate obtained in step (iii) or in step (iv), or the mix obtained in step (v), into hard capsules.

## Patentansprüche

1. Pharmazeutische Zusammensetzung, umfassend eine Granulat, umfassend einen Wirkstoff, wobei der Wirkstoff Anagrelidhydrochloridmonohydrat ist, wobei die Menge an Wirkstoff zwischen 0,42 und 1,59 Gew.-% in Bezug auf die Gesamtmenge der pharmazeutischen Zusammensetzung liegt, vorzugsweise zwischen 0,43 und 0,53 Gew.-% in Bezug auf die Gesamtmenge der pharmazeutischen Zusammensetzung, wobei sich mindestens 85%, vorzugsweise mindestens 90% des Wirkstoffs der pharmazeutischen Zusammensetzung in 15 Minuten in einer Paddelauflösungstesteinrichtung bei 50 U/min mit einer HCI-Lösung bei pH 1,2 als Auflösungsmedium und einem Volumen von 900 ml pro Gefäß auflöst, wobei die pharmazeutische Zusammensetzung einen Füllstoff umfasst, wobei der Füllstoff eine Mischung aus wasserfreier Lactose, Lactosemonohydrat und mikrokristalliner Cellulose ist; und wobei die pharmazeutische Zusammensetzung weniger als 60 mg Lactosemonohydrat umfasst.

2. Pharmazeutische Zusammensetzung nach dem vorhergehenden Anspruch, wobei die pharmazeutische Zusammensetzung ein Granulat umfasst, das den Wirkstoff enthält, wobei das Granulat eine Partikelgrößenvolumenverteilung mit einem D90 zwischen 70 und 700 Mikrometer aufweist, wenn es durch Siebanalyse gemessen wird.

3. Pharmazeutische Zusammensetzung nach einem der zwei vorhergehenden Ansprüche, wobei die pharmazeutische Zusammensetzung mindestens ein Schmiermittel umfasst, wobei das Schmiermittel vorzugsweise Magnesiumstearat ist und/oder das Schmiermittel vorzugsweise extragranular ist.

4. Pharmazeutische Zusammensetzung nach einem der vorhergehenden Ansprüche, wobei die pharmazeutische Zusammensetzung durch Nassgranulation hergestellt wird, und/oder in Form einer Hartkapsel vorliegt.

5. Pharmazeutische Zusammensetzung nach dem vorhergehenden Anspruch, wobei die Menge an Schmiermittel im Bereich von 0,3 bis 1,2 Gew.-% in Bezug auf die Gesamtmenge der pharmazeutischen Zusammensetzung liegt, vorzugsweise im Bereich von 0,4 bis 0,6 Gew.-% in Bezug auf die Gesamtmenge der pharmazeutischen Zusammensetzung liegt und/oder wobei die pharmazeutische Zusammensetzung eine Menge an Wirkstoff pro Hartkapsel zwischen 0,45 und 1,30 mg, vorzugsweise zwischen 0,48 und 1,27 mg umfasst, mehr bevorzugt die pharmazeutische Zusammensetzung eine Menge an Wirkstoff pro Hartkapsel entsprechend 0,5 mg oder 1 mg des Wirkstoffs als freie Base umfasst.

6. Pharmazeutische Charge, umfassend mindestens 20.000, vorzugsweise mindestens 50.000, mehr bevorzugt mindestens 100.000 Hartkapseln, wie in einem der Ansprüche 4 oder 5 definiert.

7. Verfahren zur Herstellung einer pharmazeutischen Zusammensetzung nach einem der Ansprüche 1 bis 4, umfassend die folgenden Schritte:
i) Wiegen, wahlweise Sieben und Mischen des Wirkstoffs und wahlweise mindestens eines pharmazeutisch verträglichen Hilfsstoffs;
ii) Nassgranulieren des in Schritt (i) erhaltenen Gemisches;
iii) Trocknen des in Schritt (ii) erhaltenen Granulats; und
iv) wahlweise Sieben des in Schritt (iii) erhaltenen Granulats.

8. Verpackte pharmazeutische Zusammensetzung in Form einer Flasche, die mindestens fünf Hartkapseln enthält, wie in einem der Ansprüche 4 bis 5 definiert, vorzugsweise wobei die Flasche lichtbeständig ist und ein Trockenmittel umfasst.

9. Pharmazeutische Zusammensetzung nach einem der Ansprüche 1 bis 5 oder die pharmazeutische Charge nach Anspruch 6 zur Verwendung bei der Behandlung von essentieller Thrombozythämie.

10. Verfahren zur Herstellung einer Hartkapsel nach einem der Ansprüche 4 oder 5,
umfassend die folgenden Schritte:
i) Wiegen, wahlweise Sieben und Mischen des Wirkstoffs und wahlweise mindestens eines pharmazeutisch verträglichen Hilfsstoffs;
ii) Nassgranulieren des in Schritt (i) erhaltenen Gemisches;
iii) Trocknen des in Schritt (ii) erhaltenen Granulats;
iv) wahlweise Sieben des in Schritt (iii) erhaltenen Granulats;
v) wahlweise Zugeben mindestens eines extragranulären pharmazeutisch verträglichen Hilfsstoffs zu dem in Schritt (iii) oder (iv) erhaltenen Granulat; und
vi) Füllen des in Schritt (i) erhaltenen Gemisches oder des in Schritt (iii) oder in Schritt (iv) erhaltenen Granulats oder des in Schritt (v) erhaltenen Granulats in Hartkapseln.

## Revendications

1. Composition pharmaceutique comprenant un granulé comprenant un agent actif, dans laquelle l'agent actif est le chlorhydrate d'anagrélide monohydraté, dans laquelle la quantité d'agent actif est entre 0,42 et 1,59 % en poids par rapport à la quantité totale de la composition pharmaceutique, de préférence entre 0,43 et 0,53 % en poids par rapport à la quantité totale de la composition pharmaceutique, dans laquelle au moins 85 %, de préférence au moins 90 % de l'agent actif de la composition pharmaceutique se dissout en 15 minutes dans un appareil de test de dissolution à pales, à 50 tr/min avec une solution d'HCl à pH 1,2 comme milieu de dissolution et un volume de 900 ml par récipient, dans laquelle la composition pharmaceutique comprend une charge, dans laquelle la charge est un mélange de lactose anhydre, de lactose monohydraté et de cellulose microcristalline ; et dans laquelle la composition pharmaceutique comprend moins de 60 mg de lactose monohydraté.

2. Composition pharmaceutique selon la revendication précédente, dans laquelle la composition pharmaceutique comprend un granulé qui contient l'agent actif, dans laquelle ledit granulé a une distribution du volume de la taille des particules avec un D90 compris entre 70 et 700 microns, lorsqu'il est mesuré par analyse par tamisage.

3. Composition pharmaceutique selon l'une quelconque des deux revendications précédentes, dans laquelle la composition pharmaceutique comprend au moins un lubrifiant, de préférence le lubrifiant est le stéarate de magnésium et/ou le lubrifiant est de préférence extragranulaire.

4. Composition pharmaceutique selon l'une quelconque des revendications précédentes, dans laquelle la composition pharmaceutique est préparée par granulation humide et/ou est sous la forme d'une gélule.

5. Composition pharmaceutique selon la revendication précédente, dans laquelle la quantité de lubrifiant va de 0,3 à 1,2 % en poids par rapport à la quantité totale de la composition pharmaceutique, varie de préférence de 0,4 à 0,6 % en poids par rapport à la quantité totale de la composition pharmaceutique, et/ou dans laquelle la composition pharmaceutique comprend une quantité d'agent actif par gélule entre 0,45 et 1,30 mg, de préférence entre 0,48 et 1,27 mg, plus préférablement la composition pharmaceutique comprend une quantité d'agent actif par gélule équivalente à 0,5 mg ou 1 mg de l'agent actif sous forme de base libre.

6. Lot pharmaceutique comprenant au moins 20 000, de préférence au moins 50 000, plus préférablement au moins 100 000 gélules telles que définies dans l'une quelconque des revendications 4 ou 5.

7. Procédé de préparation d'une composition pharmaceutique selon les revendications 1 à 4, comprenant les étapes suivantes :
i) peser, éventuellement tamiser et mélanger l'agent actif et éventuellement au moins un excipient pharmaceutiquement acceptable ;
ii) granuler par voie humide le mélange obtenu à l'étape (i) ;
iii) sécher le granulé obtenu à l'étape (ii) ; et
iv) éventuellement tamiser le granulé obtenu à l'étape (iii).

8. Composition pharmaceutique emballée sous forme de flacon contenant au moins cinq gélules telles que définies dans l'une quelconque des revendications 4 à 5, de préférence dans laquelle ledit flacon est résistant à la lumière et comprend un déshydratant.

9. Composition pharmaceutique selon l'une quelconque des revendications 1 à 5 ou lot pharmaceutique selon la revendication 6 destinée à être utilisée dans le traitement de la thrombocythémie essentielle.

10. Procédé de fabrication d'une gélule selon l'une quelconque des revendications 4 ou 5,
comprenant les étapes suivantes :
i) peser, éventuellement tamiser et mélanger l'agent actif et éventuellement au moins un excipient pharmaceutiquement acceptable ;
ii) granuler par voie humide le mélange obtenu à l'étape (i) ;
iii) sécher le granulé obtenu à l'étape (ii) ;
iv) éventuellement tamiser le granulé obtenu à l'étape (iii) ;
v) éventuellement ajouter au moins un excipient extragranulaire pharmaceutiquement acceptable au granulé obtenu à l'étape (iii) ou (iv) ; et
vi) remplir le mélange obtenu à l'étape (i), ou le granulé obtenu à l'étape (iii) ou à l'étape (iv), ou le mélange obtenu à l'étape (v), dans des gélules.
